# EUROPEAN PATENT SPECIFICATION

(11) **EP 1 197 184 B1**
(45) Date of publication and mention of the grant of the patent: **27.04.2005**
(21) Application number: 98921830.0
(22) Date of filing: 27.05.1998
(51) Int. Cl.: A61B 18/14

(54) **APPARATUS FOR BIOLOGICAL TISSUE TREATMENT UTILIZING HIGH FREQUENCY**
VORRICHTUNG ZUR HOCHFREQUENZ-BEHANDLUNG VON BIOLOGISCHEM GEWEBE
APPAREIL POUR LE TRAITEMENT HAUTE FREQUENCE DE TISSUS BIOLOGIQUES

(30) Priority: 02.07.1997 JP 19180297
(43) Date of publication of application: 17.04.2002
(73) Proprietor: Kabushikikaisha Nihon M.D.M, Tokyo 162-0066 (JP)
(72) Inventor: WATANABE, Yasuo Kabushikikaisha Nihon M. D. M, Tokyo 162-0066 (JP); ZHAO, Wei Kabushikikaisha Nihon M. D. M, Tokyo 162-0066 (JP)
(74) Representative: Säger, Manfred
(86) International application number: PCT/JP1998/002323
(87) International publication number: WO 1999/001077

(56) References cited:
- EP-A- 0 752 235
- JP-A- 6 000 191
- JP-A- 6 209 952
- JP-A- 7 255 738
- US-A- 3 875 945
- US-A- 4 580 562
- US-A- 5 743 900

## Description

### Technical Field

The present invention relates to an organism tissue treatment apparatus using a radio frequency that exposes an organism tissue in an electromagnetic field adjacent to a tip of an electromagnetic wave radiant electrode to radiate a strong electromagnetic wave from the tip without a diffusion electrode, whereby a treatment including an evaporation, dissection, coagulation, hemostasis, and the like can be applied to a local region of the organism tissue.

### Background Art

Prior art document JP-A-07 255738 discloses an apparatus for radio frequency treatment of living tissue without the use of a neutral electrode. The radio frequency current is supplied to an electrode from a radio frequency generator via a coaxial cable and a matching unit. However, the matching unit according to prior art document JP-A-07 255738 is mounted inside the probe holding the electrode.

Prior art document US-A-4 580 562 reveals an electrosurgical apparatus for radio frequency treatment of living tissue; similar to prior art document JP-A-07 255738, in the apparatus according to prior art document US-A-4 580 562 the matching unit is mounted inside the probe

Prior art document US-A-3 875 945 discloses an apparatus for radio frequency treatment of living tissue in which the matching unit is arranged outside the probe. The electromagnetic energy serves to generate an arc discharge from the electrode tip for treatment of a zone in the living tissue.

Prior art document US-A-5 464 405 (= JP-A-06 209952) reveals bipolar electric surgical tweezers comprising two electrically conductive arms and a perfusion connection port, having the axis of the electrode grip at an offset position from the axis of the electrode to acquire a good operator's visual field.

Prior art document JP-A-06 000191 discloses a special catheter which is constituted to transmit high-frequency energy outputted from a high frequency generator to the lesion within the heart. The coaxial cable according to prior art document JP-A-06 000191 is divided in two divided cables connected to each other by a connector.

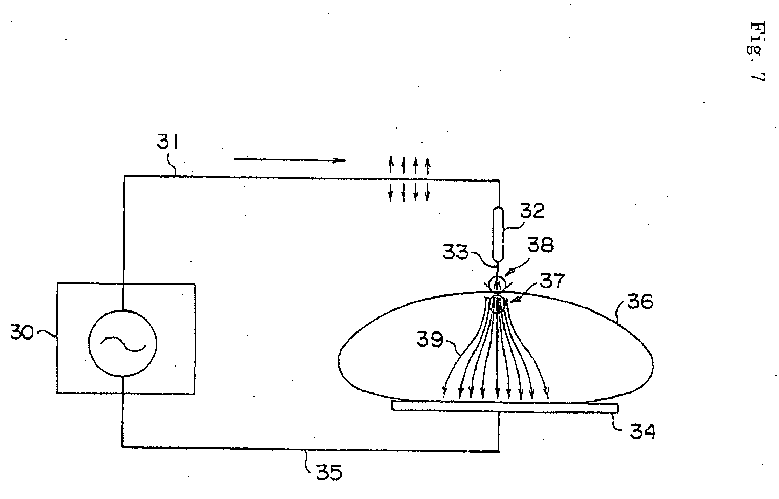

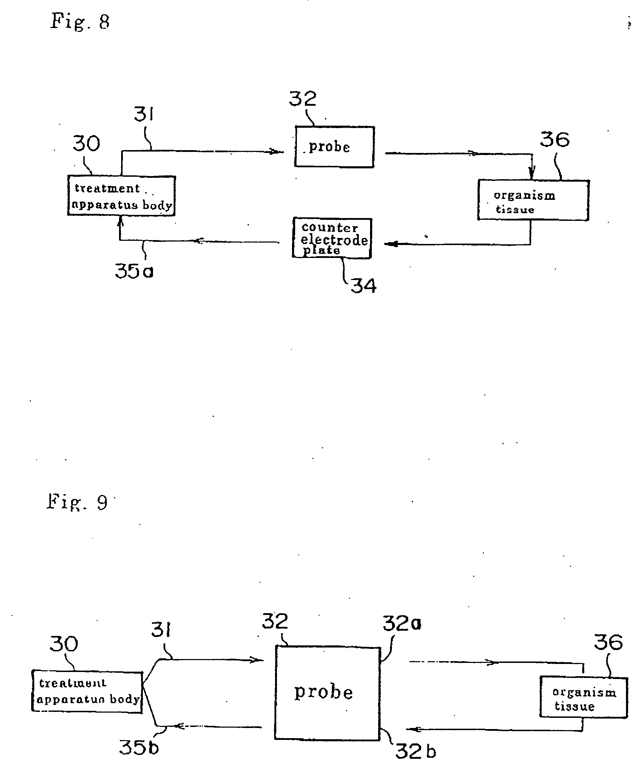

Fig. 7 illustrates a conceptual construction of a conventional organism tissue treatment apparatus using a radio frequency, Fig. 8 illustrates a transmission path of a radio frequency current when a mono-polar probe is used in the organism tissue treatment apparatus using a radio frequency in Fig. 7, and Fig. 9 illustrates the transmission path of the radio frequency current when a bi-polar probe is used in the organism tissue treatment apparatus using a radio frequency in Fig. 7.

First, as shown in Fig. 7, when applying a treatment such as a dissection to an organism tissue 36 by using the conventional organism tissue treatment apparatus using a radio frequency, usually the operator uses a discharge electrode 33 projecting from the tip of a probe 32, as a radio knife, for example, places a counter electrode plate 34 also called as a diffusion electrode located on the opposite side of the discharge electrode 33 so as to put the organism tissue 36 therebetween, connects the discharge electrode 33 through a radio frequency current cable 31 to one output electrode of a treatment apparatus body 30 provided with a radio frequency power source that generates a radio frequency covering 0.3 MHz to 5.0 MHz, and connects the counter electrode plate 34 through a radio frequency current cable 35 to the other output electrode of the treatment apparatus body 30.

And, the radio frequency current is flown in the discharge electrode 33 to generate a discharge from a discharge part 38 between the discharge electrode 33 and the organism tissue 36. The local heat generated by the discharge thereat and the Joule heat generated by the electric resistance of the organism tissue 36 to the current flown concentratedly into the organism tissue 36 from the discharge part 38 heat and cauterize the organism tissue 36 locally, whereby a treatment such as a dissection has been carried out to the local region of the organism tissue 36. Thereat, a current 39 flown into the organism tissue 36 from the discharge electrode 33 flows through the organism tissue 36 to the counter electrode plate, namely, the diffusion electrode 34 spread in a sheet. The current flown into the diffusion electrode 34 returns to the treatment apparatus body 30 through the radio frequency current cable 35.

Fig. 8 illustrates a transmission path of a radio frequency current when a mono-polar probe is used as the probe 32 of the organism tissue treatment apparatus using a radio frequency shown in Fig. 7. In Fig. 8, the radio frequency current transmitted to the probe 32 from the treatment apparatus body 30 through the radio frequency current cable 31 of a single core flows into the organism tissue 36 by a discharge between the discharge electrode 33 and the organism tissue 36. Further, the radio frequency current runs across the organism tissue 36 into the counter electrode plate 34, and returns to the treatment apparatus body 30 from the counter electrode plate 34 through a radio frequency current cable 35a of a single core or multiple core. That is, the radio frequency current cable 31, probe 32, organism tissue 36, counter electrode plate 34, and radio frequency current cable 35a form a closed circuit.

Fig. 9 illustrates a construction different from the foregoing Fig. 8, which illustrates a transmission path of a radio frequency current when a bi-polar probe is used as the probe 32 of the organism tissue treatment apparatus using a radio frequency shown in Fig. 7. In Fig. 9, the radio frequency current transmitted to the probe 32 from the treatment apparatus body 30 through the radio frequency current cable 31 of a single core flows into the organism tissue 36 by a discharge between the organism tissue 36 and one discharge electrode of a pair of the discharge electrodes 33, connected to an active electrode 32a of the probe 32. Further, the radio frequency current flows into the other discharge electrode of the foregoing discharge electrode 33, connected to an passive electrode 32b of the probe 32, from the organism tissue 36, further flows into the probe 32 from the other discharge electrode connected to the passive electrode 32b through the passive electrode 32b of the probe 32, and further returns to the treatment apparatus body 30 from the probe 32 through a radio frequency current cable 35b of a single core. That is, the treatment apparatus body 30, radio frequency current cable 31, active electrode 32a of the probe 32, organism tissue 36, passive electrode 32b of the probe 32, and radio frequency current cable 35b form a closed circuit.

In Fig. 7, when the radio frequency current of 0.3 MHz to 5.0 MHz is supplied to the discharge electrode 33, as mentioned above, the current 39 flows from the discharge part 38 across the organism tissue 36 to the diffusion electrode 34 spread in a sheet. At this moment, the heat propagated by the thermal conduction from a treated part such as a dissection, locally heated by the discharge, and the Joule heat generated by the electric resistance of the organism tissue 36 to the current flown concentratedly into the organism tissue 36 from the discharge part 38 will denature a periphery of the treated part of the organism tissue 36 greatly, covering a considerably large area.

And, in the conventional organism tissue treatment apparatus using a radio frequency, the counter electrode plate 34 as the diffusion electrode is indispensable, which involves a necessity of the radio frequency current cable 35 for connecting the counter electrode plate 34 to the treatment apparatus body 30. Besides, in a treatment such as a dissection to the organism tissue 36, the radio frequency current flows across the organism tissue 36 between the discharge electrode 33 and the counter electrode plate 34, and there emerge fears over various adverse effects to the organism tissue 36 due to this across current.

Thus, in the conventional organism tissue treatment apparatus using a radio frequency, the frequency band in the actual use covers 0.3 MHz to 5.0 MHz, which is considerably low as a radio frequency, and the counter electrode plate as the diffusion electrode is indispensable in order to form a closed radio frequency circuit, including the organism tissue in the circuit. And, since the treatment to the organism tissue is carried out simply by utilizing the local heat by the discharge between the discharge electrode and the organism tissue and the Joule heat by the current running through the organism tissue, there is a limit to enhance the concentration of the heating energy in the local region, namely, the treated part of the organism tissue; and therefore, when a treatment such as a dissection is applied to the local region of the organism tissue, there appears an inadequacy that greatly denatures a periphery of the treated part of the organism tissue, covering a considerably large area. Further, since the radio frequency current flows across the organism tissue between the discharge electrode and the counter electrode plate, there remain fears over various adverse effects to the organism tissue due to this across current.

Accordingly, the invention intends to provide an organism tissue treatment apparatus using a radio frequency that employs the radio frequency current of a higher frequency than the frequency band used in the conventional organism tissue treatment apparatus using a radio frequency, whereby an electromagnetic wave energy generated by the radio frequency current can be used effectively, and a radio frequency closed circuit including an organism tissue inside thereof is not needed to be formed, as it is indispensable in the conventional technique, therefore, such a counter electrode plate as the diffusion electrode becomes needless; and, the organism tissue treatment apparatus effectively uses the electromagnetic wave energy to concentrate a local heat by a discharge between a discharge electrode and an organism tissue and a Joule heat by a current running locally through the organism tissue in a local region of the organism tissue, whereby a treatment to the organism tissue can precisely be applied, and as a result, a treatment to the local region of the organism tissue can be carried out without widely denaturing a periphery of the treated part of the organism tissue, without a radio frequency current flowing across the organism tissue, and various treatments to the organism tissue can securely be applied.

Further, the invention intends to provide an organism tissue treatment apparatus using a radio frequency that prevents a leakage of the electromagnetic wave from a radio frequency current cable to thereby enhance the transmission efficiency of the radio frequency energy, whereby the radio frequency current cable is able to effectively demonstrate the matching function as a matching cable.

Further, while making a matching adjustment covering the whole length of the matching cable, the invention on one side decreases the electric resistance of the cable on the main body of the organism tissue treatment apparatus using a radio frequency as low as possible so as to restrain the energy loss of the radio frequency current as low as possible, and on the other side enhances the flexibility of the cable on the side of the tip and enhances the capability to follow a probe during handling the probe to enhance the maneuverability of the probe.

Further, the invention intends to provide an organism tissue treatment apparatus using a radio frequency that, in the foregoing probe, an electromagnetic wave radiated from an active output terminal and an electromagnetic wave radiant electrode is captured by a passive output terminal, and thereby, the diffusion of a radio frequency energy into the air can be prevented so as to effectively recover the radio frequency energy.

Further, the invention intends to provide an organism tissue treatment apparatus using a radio frequency that, in the foregoing probe, the radio frequency characteristic of the radio frequency current flowing through an electromagnetic wave radiant electrode can be varied in accordance with the construction of an impedance circuit between the electromagnetic wave radiant electrode and the passive output terminal, and an optimum radio frequency characteristic can be selected in accordance with the requirements according to the treatment to the organism tissue.

Further, the invention intends to provide an organism tissue treatment apparatus using a radio frequency that a treatment including an evaporation and dissection, etc., can be applied to the local region of the organism tissue, and a treatment including a coagulation and hemostasis, etc., can effectively be applied by utilizing a thermal denaturation generated in the local region of the organism tissue.

Further, the invention intends to provide a probe that, when a treatment is applied to the organism tissue using the foregoing organism tissue treatment apparatus using a radio frequency, the grip of the probe or the finger of an operator holding the grip does not obstruct the line of sight from the backward on the axis of the foregoing electromagnetic wave radiant electrode to thereby secure a safe operation and a precise treatment to the organism tissue.

### Disclosure of the Invention

The organism tissue treatment apparatus of the invention is defined by claim 1.

Preferred embodiments are defined in the dependent claims.

### Brief Description of the Drawings

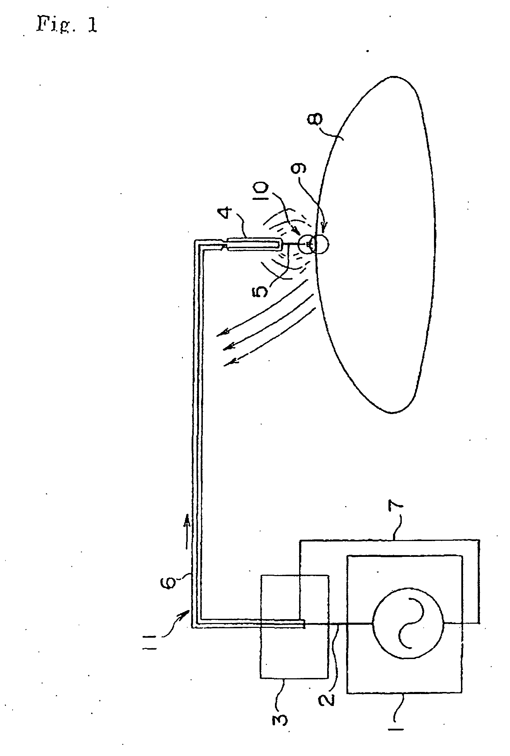

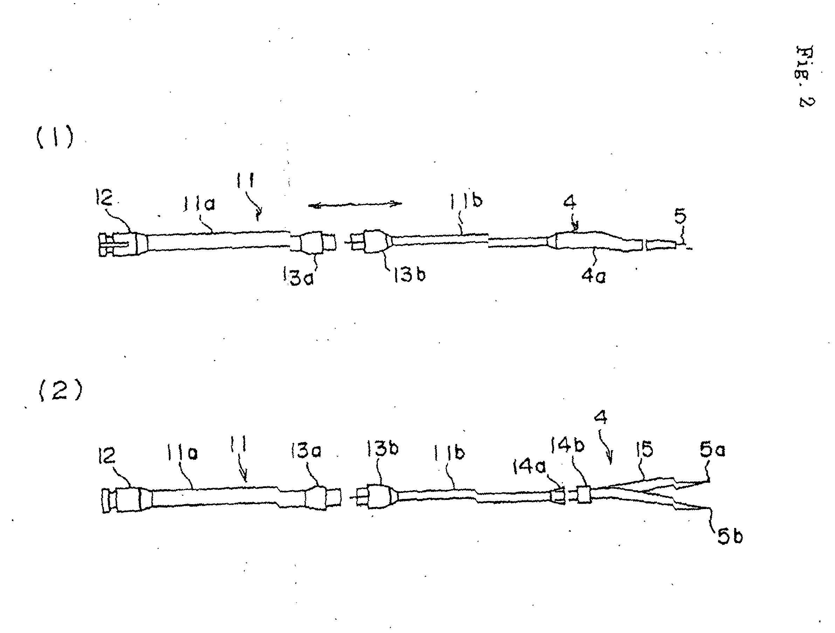

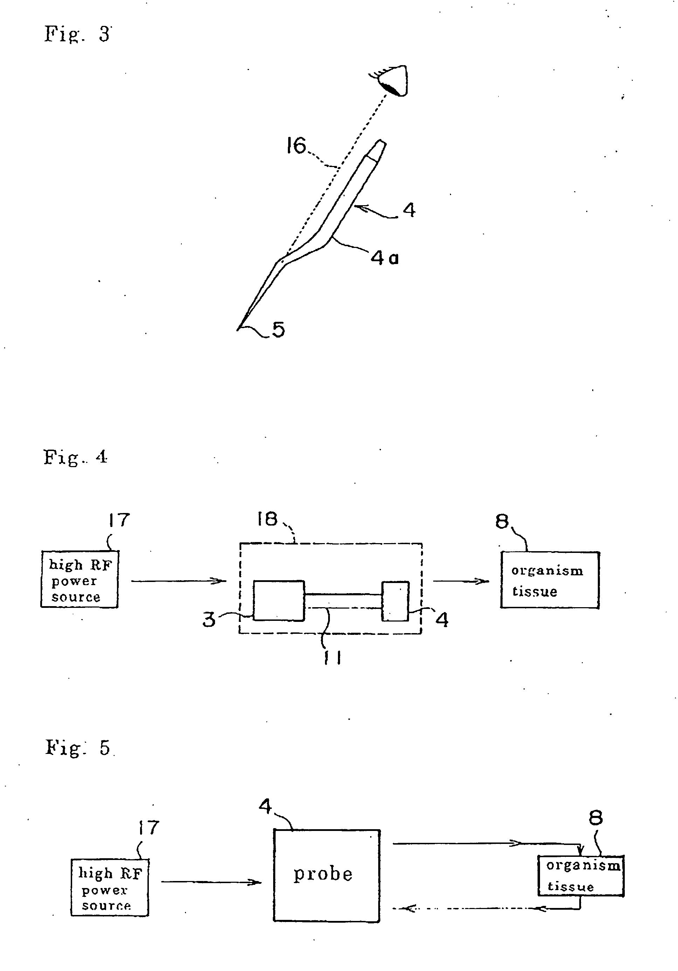

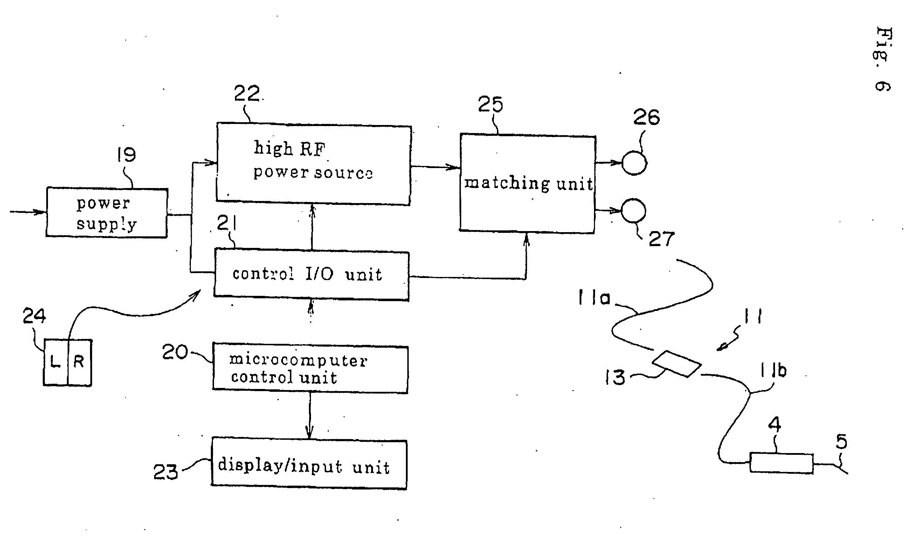

Fig. 1 illustrates a conceptual construction of an organism tissue treatment apparatus of the invention; Fig. 2 (1) is a side view to illustrate one example of a matching cable and a mono-polar probe that can be used in the organism tissue treatment apparatus of the invention; Fig. 2 (2) is a side view to illustrate one example of a matching cable and a bi-polar probe different from those in Fig. 2 (1) used in the organism tissue treatment apparatus of the invention; Fig. 3 is a side view to explain a state in which the mono-polar probe in Fig. 2 (1) is used; Fig. 4 illustrates a transmission path of a radio frequency current and an electromagnetic wave energy when the mono-polar probe is used in the organism tissue treatment apparatus of the invention; Fig. 5 illustrates a transmission path of a radio frequency current and an electromagnetic wave energy when the bi-polar probe is used in the organism tissue treatment apparatus of the invention; and Fig. 6 illustrates a transmission path of a current and a control signal from power supply of the organism tissue treatment apparatus of the invention.

### Best Mode for Carrying out the Invention

In Fig. 1, the organism tissue treatment apparatus of the invention contains a high RF (Radio Frequency) power source 1 that generates a radio frequency current of a high band radio frequency covering 8 MHz to 60 MHz, or a higher frequency than that. This high RF power source 1 supplies an electromagnetic wave radiant electrode 5 with the foregoing radio frequency current through a matching unit 3 and a matching cable 11, and the electromagnetic wave radiant electrode 5 is held by the grip of a probe 4 formed of an insulating material. The tip of the electromagnetic wave radiant electrode 5 projects still forwarder from the front end of the grip of the probe 4.

The matching cable 11 has a core wire 2 and a shielded wire 6 that coaxially encloses the core wire 2 through the insulating material. The base terminal side of the core wire 2 is connected to one electrode of the high RF power source 1 through the matching unit 3, and the base terminal side of the shielded wire 6 is connected to the other electrode of the high RF power source 1 through the matching unit 3 and a lead wire 7. The front end side of the core wire is connected to the electromagnetic wave radiant electrode 5 held by the grip of the probe 4. The shielded wire 6 encloses the core wire 2 from the matching unit 3 to a position near the tip of the electromagnetic wave radiant electrode 5 inside the probe 4.

Thus, since the shielded wire 6 encloses the core wire 2 from the matching unit 3 to the position near the tip of the electromagnetic wave radiant electrode 5 inside the probe 4, when a radio frequency current is transmitted from the high RF power source 1 to the electromagnetic wave radiant electrode 5 through the core wire 2, this shielded wire 6 effectively capture the electromagnetic wave radiated from the core wire 2. As a result, the energy radiated as an electromagnetic wave from the core wire 2 is prevented from dissipating into the external system as the air on the way from the matching unit 3 to the electromagnetic wave radiant electrode 5 held by the probe 4. And at the same time, the matching cable 11 with its length and diameter adjusted cooperates with the matching unit 3 so as to exert a good matching function.

As mentioned above, when the electromagnetic wave radiant electrode 5 is supplied with a radio frequency current of a high band radio frequency covering 8 MHz to 60 MHz, or a higher frequency than that from the high RF power source 1 through the matching unit 3 and the matching cable 11, the electromagnetic wave radiant electrode 5 radiates a strong electromagnetic wave from its tip.

And, when the electrode tip of the electromagnetic wave radiant electrode 5 is approached to a local region 9 of an organism tissue 8, until the organism tissue is exposed in the electromagnetic field near the electrode tip of the electromagnetic wave radiant electrode 5, a discharge part 10 between the electrode tip of the electromagnetic wave radiant electrode 5 and the local region 9 of the organism tissue 8 generates an arc discharge. And at the same time, an arc current flows into the local region 9 of the organism tissue 8, serving the organism tissue 8 as the earth, to locally generate a Joule heat. By utilizing the electromagnetic energy of the electromagnetic field near the electrode tip of the electromagnetic wave radiant electrode 5 under the effect of the discharge energy accompanied with the arc discharge generated at the discharge part 10 between the electrode tip of the electromagnetic wave radiant electrode 5 and the local region 9 of the organism tissue 8, servirig the organism tissue 8 as the earth, and the Joule heat locally generated by the current flowing into the local region 9 of the organism tissue 8, serving the organism tissue 8 as the earth, a treatment including an evaporation, dissection, and a coagulation and hemostasis by the thermal denaturation, etc., can be applied to the local region 9 of the organism tissue 8.

As mentioned above, in the organism tissue treatment apparatus using a radio frequency of the invention, when the tip of the electromagnetic wave radiant electrode 5 is approached to the local region 9 of the organism tissue 8, until the organism tissue is exposed in the electromagnetic field near the tip of the electromagnetic wave radiant electrode 5, the discharge part 10 between the tip of the electromagnetic wave radiant electrode 5 and the local region 9 of the organism tissue 8 generates an arc discharge, and at the same time, an arc current flows into the local region 9 of the organism tissue 8, serving the organism tissue 8 as the earth; and therefore, the diffusion electrode or the counter electrode plate 34 shown in Fig. 7 become completely needless, which is indispensable in the conventional organism tissue treatment apparatus using a radio frequency.

And, the radio frequency current is directly supplied to the electromagnetic wave radiant electrode 5 in the probe 4 through the radio frequency current cable 11 from the high RF power source 1 as the radio frequency power supply means.

Fig. 2 (1) illustrates one example of a radio frequency current cable having a matching function, namely, a matching cable and a mono-polar probe used in the organism tissue treatment apparatus of the invention. In Fig. 2 (1), the matching cable 11 is divided into a plurality of cables, for example, two divided cables 11a and 11b as shown in the drawing, on the intermediate position of the matching cable 11 through a connector formed of connector sockets 13a and 13b which can be engaged and detached each other. And, of the each adjoining divided cables 11a and 11b, the divided cable on the side of the tip, namely, the divided cable 11b connected integrally to the probe 4 with a grip 4a formed, as shown in the drawing, is made smaller in the cable diameter than the divided cable on the main body of the organism tissue treatment apparatus using a radio frequency, namely, the divided cable 11a connected to the main body of the organism tissue treatment apparatus using a radio frequency through a connector socket 12 as shown in the drawing.

In this case, as shown in Fig. 2 (1), instead of integrally connecting the probe 4 to the divided cable 11b, it is arranged to connect the probe 4 and the divided cable 11b through a connector so as to connect various types of probes detachably to the front end of the divided cable 11b. Further, it may be arranged to connect the electromagnetic wave radiant electrode 5 having various types of tips replaceably and detachably to the active output terminal inside the probe 4.

Next, Fig. 2 (2) illustrates one example of a matching cable and a bi-polar probe different from those in Fig. 2 (1) used in the organism tissue treatment apparatus of the invention. The matching cable 11 shown in Fig. 2 (2) is also divided into a plurality of cables, for example, two divided cables 11a and 11b as shown in the drawing, on the intermediate position of the matching cable 11 through a connector formed of connector sockets 13a and 13b which can be engaged and detached each other. And, of the each adjoining divided cables 11a and 11b, the divided cable on the side of the tip, namely, the divided cable 11b connected detachably to the probe 4 through a connector formed of connector sockets 14a and 14b, as shown in the drawing, is made smaller in the cable diameter than the divided cable on the main body of the organism tissue treatment apparatus using a radio frequency, namely, the divided cable 11a connected to the main body of the organism tissue treatment apparatus using a radio frequency through the connector socket 12 as shown in the drawing.

The probe 4 shown in Fig. 2 (2) is a bi-polar type probe that has a tip 5a of the electromagnetic wave radiant electrode 5 connected to the active output terminal inside the probe 4, and a tip 5b of the facing electrode equipped to face to this electromagnetic wave radiant electrode, connected to the passive output terminal inside the probe 4. In such a bi-polar type probe, various types of the probes 4 can detachably be connected to the front end of the divided cable 11b. And, it may be arranged to connect the electromagnetic wave radiant electrode 5 having various types of tips replaceably and detachably to the active output terminal inside the probe 4.

As illustrated in Fig. 2 (1) and Fig. 2 (2), the matching cable 11 is divided into a plurality of cables through a connector, and the divided cable 11b on the side of the tip is made smaller in the cable diameter than the divided cable 11a on the main body of the organism tissue treatment apparatus using a radio frequency of the each adjoining divided cables, thereby making a matching adjustment covering the whole length of the matching cable 11. And on one side, it becomes possible to decrease the electric resistance of the divided cable 11a on the main body of the organism tissue treatment apparatus using a radio frequency as low as possible, and to reduce the energy loss of the radio frequency current as low as possible. And on the other side, it becomes possible to increase the flexibility of the divided cable 11b on the side of the tip, to expand the capability to follow the probe 4 during handling the probe 4, and to enhance the maneuverability of the probe 4.

By making the grip 4a of the probe 4 in a bent form, as shown in Fig. 3, the position of the axis of the grip 4a of the probe 4 relative to the electromagnetic wave radiant electrode 5 is set at a relatively offset position from the axis of the electromagnetic wave radiant electrode 5, so that the grip 4a or the finger of an operator holding the grip 4a does not obstruct a line of sight 16 on the axis of the electromagnetic wave radiant electrode 5 viewing from the backward of the grip 4a. Thus, the operator is able to maneuver the probe 4 precisely and securely without the line of sight 16 on the axis of the electromagnetic wave radiant electrode 5 being obstructed by the grip 4a or the finger of the operator.

The probe 4 contains the active output electrode and the passive output electrode inside the grip 4a, and the active output electrode is connected the electromagnetic wave radiant electrode 5. As the tip shape of the electromagnetic wave radiant electrode 5, a sharp tip such as a needle type or a blade type and an obtuse tip such as ball type less than a limited size or a ring type can be adopted, and the tip of an optimum shape can be selected for use in accordance with the treatment to the organism tissue.

Fig. 4 illustrates a transmission path of a radio frequency current and an electromagnetic wave energy when the mono-polar probe is used in the organism tissue treatment apparatus using a radio frequency in Fig. 1. In Fig. 4, a radio frequency current of at least a high band radio frequency generated by a high RF power source 17, a frequency covering 8 MHz to 60 MHz, or a higher frequency than that is supplied to an energy converter 18 including the matching unit 3, the matching cable 11, and the probe 4; and thereby, the electromagnetic wave radiant electrode 5 of the probe 4 radiates a strong electromagnetic wave from the tip thereof.

And, when the electrode tip of the electromagnetic wave radiant electrode 5 is approached to a local region 9 of an organism tissue 8, until the organism tissue 8 is exposed in the electromagnetic field near the electrode tip of the electromagnetic wave radiant electrode 5, a discharge part 10 between the tip of the electromagnetic wave radiant electrode 5 and the local region 9 of the organism tissue 8 generates an arc discharge. And at the same time, an arc current flows into the local region 9 of the organism tissue 8, serving the organism tissue 8 as the earth, to locally generate a Joule heat. By utilizing the electromagnetic energy of the electromagnetic field near the electrode tip of the electromagnetic wave radiant electrode 5, under the effect of the discharge energy accompanied with the arc discharge generated at the discharge part 10 between the tip of the electromagnetic wave radiant electrode 5 and the local region 9 of the organism tissue 8, serving the organism tissue 8 as the earth, and the Joule heat locally generated by the current flowing into the local region 9 of the organism tissue 8, serving the organism tissue 8 as the earth, a treatment including an evaporation, dissection, and a coagulation and hemostasis by the thermal denaturation, etc., can be applied to the local region 9 of the organism tissue 8.

In contrast to this, Fig. 5 illustrates a transmission path of a radio frequency current and an electromagnetic wave energy when the bi-polar probe is used in the organism tissue treatment apparatus using a radio frequency in Fig. 1. In Fig. 5, a radio frequency current of at least a high band radio frequency generated by the high RF power source 17, a frequency covering 8 MHz to 60 MHz, or a higher frequency than that is supplied to the probe 4 through a coaxial cable.

Thus, in the bi-polar probe, the electromagnetic wave radiant electrode and the facing electrode that makes a pair with the electromagnetic wave radiant electrode to form a bi-polar electrode together with the electromagnetic wave radiant electrode are held by the grip of the probe, and both of the tips of the electromagnetic wave radiant electrode and the facing electrode are projected from the front end of the grip; and inside the grip, the foregoing electromagnetic wave radiant electrode is connected to the active output terminal, and on the other hand, the foregoing facing electrode is connected to the passive output terminal.

In the bi-polar probe, in the same manner as in the mono-polar probe, the electromagnetic wave radiant electrode radiates a strong electromagnetic wave from the tip thereof. And, when the tip of the electromagnetic wave radiant electrode is approached to the local region 9 of the organism tissue 8, until the organism tissue 8 is exposed in the electromagnetic field near the tip of the electromagnetic wave radiant electrode, a discharge part 10 between the tip of the electromagnetic wave radiant electrode and the local region 9 of the organism tissue 8 generates an arc discharge. And at the same time, an arc current flows into the tip of the facing electrode through the organism tissue 8, to generate a local Joule heat in the local region 9 of the organism tissue 8. As a result, a treatment such as an evaporation and dissection, etc., can be applied to the local region of the organism tissue, and a treatment such as a coagulation and hemostasis, etc., can be applied by utilizing the thermal denaturation generated in the local region 9 of the organism tissue 8.

In the foregoing mono-polar probe 4, the electromagnetic wave radiant electrode 5 is held by the grip 4a of the probe 4, and the tip of the electromagnetic wave radiant electrode 5 is projected from the front end of the grip 4a. Inside the grip 4a, the electromagnetic wave radiant electrode 5 is connected to the active output terminal, however the passive output terminal can be disconnected from the circuit and be maintained in the open state. In the bi-polar probe, however, the electromagnetic wave radiant electrode is connected to the active output terminal, and on the other hand, the facing electrode may be connected to the passive output terminal.

Further, in the same mono-polar probe 4, in which the electromagnetic wave radiant electrode 5 is held by the grip 4a of the probe 4, the electrode tip side of the electromagnetic wave radiant electrode 5 is projected from the front end of the grip 4a, and the electromagnetic wave radiant electrode 5 is connected to the active output terminal inside the grip 4a, it can be arranged that the electromagnetic wave radiant electrode 5 is connected to the passive output terminal through an impedance circuit including at least one of at least one capacitor and at least one inductor. In this case, since the radio frequency characteristic of the radio frequency current flowing through the electromagnetic wave radiant electrode 5 can be varied in accordance with the construction of the impedance circuit between the electromagnetic wave radiant electrode 5 and the passive output terminal, the optimum radio frequency characteristic can be selected in accordance with the requirements according to the treatment to the organism tissue 8. In the bi-polar probe in the same manner as in the mono-polar probe, in case of a probe such that the electromagnetic wave radiant electrode is connected to the active terminal, it can be arranged that the electromagnetic wave radiant electrode is connected to the passive terminal through an impedance circuit including at least one of at least one capacitor and at least one inductor.

Fig. 6 illustrates one example of a transmission path of a current and a control signal from a power supply of the organism tissue treatment apparatus using a radio frequency to the probe in Fig. 1. In Fig. 6, a power supply 19 may be a usual commercial power supply, and a current taken in the power supply 19 is converted into a radio frequency current of a high band radio frequency covering 8 MHz to 60 MHz, or a higher frequency than that by a high RF power source 22. The radio frequency current generated by the high RF power source 22 is transmitted to a matching unit 25. The matching unit 25 has a mono-polar output unit 26 and a bi-polar output unit 27.

A microcomputer control unit 20 executes an output control of the high RF power source 22 and a matching control of the matching unit 25 through a control I/O unit 21, and renders a display/input unit 23 to display necessary items relating to the output state of the high RF power source 22 and the matching operation state of the matching unit 25, and the like. To connect a foot switch or pedal switch 24 to the control I/O unit 21 and to press this foot switch or pedal switch 24 will connect or disconnect the output of the matching unit 25.

The probe 4 is connected to the matching unit 25 through the matching cable 11 including the divided cable 11a of a larger diameter, a relay connector 13, and the divided cable 11b of a smaller diameter. Here, in case the probe 4 having the electromagnetic wave radiant electrode 5 is a mono-polar type, the matching cable 11 is connected to the mono-polar output unit 26 of the matching unit 25; and in case the probe 4 is a bi-polar type, the matching cable 11 is connected to the bi-polar output unit 27 of the matching unit 25.

To enhance the effect of a treatment to an organism tissue, the output wave form of a radio frequency current can be reshaped and outputted. When a bi-polar probe is used, for example, the duty cycle of a modulation pulse wave can be adjusted in order to prevent burnt deposits on the tip of the electromagnetic wave radiant electrode 5. In case the probe of a bi-polar type is used, the duty cycle of a modulation pulse wave can be adjusted in accordance with a wide-spreading hemostasis and a narrow hemostasis. In case the main frequency is 13.56 MHz of a continuous sine wave, for example, this continuous sine wave can be modulated by a modulation pulse wave of 100 kHz - 300 kHz to reshape the output wave form. Moreover, the output wave form can be reshaped by adjusting the duty cycle of this modulation pulse wave of 100 kHz - 300 kHz.

By adjusting the duty cycle of a modulation pulse wave, the following four modes, for example, can be switched.

| | |
|---|---|
| mode of evaporation and dissection | duty cycle 100 % |
| mode of dissection and hemostasis (mixture 1) | duty cycle 85 - 95 % |
| mode of dissection and hemostasis (mixture 2) | duty cycle 75 - 85 % |
| mode of hemostasis (coagulation) | duty cycle 30 - 45 % |

### Industrial Applicability

The organism tissue treatment apparatus using a radio frequency of the invention will achieve the following effects.
**(1)** The organism tissue treatment apparatus of the invention has the electromagnetic wave radiant electrode held by the grip of the probe, which is supplied with a radio frequency current having a high band radio frequency, and thereby radiates a strong electromagnetic wave from the tip thereof, and in a state that an organism tissue is exposed in an electromagnetic field adjacent to the foregoing tip of the electromagnetic wave radiant electrode, the organism tissue treatment apparatus is designed so that a treatment including an evaporation, dissection, coagulation, hemostasis, and the like can be applied to the foregoing organism tissue by the probe under the effect of an arc discharge generated between the foregoing tip of the electromagnetic wave radiant electrode and a local region of the foregoing organism tissue and a Joule heat locally generated by a current flown into the organism tissue as the earth. Therefore, while employing the radio frequency current of a higher frequency than the frequency band used in the conventional organism tissue treatment apparatus, the organism tissue treatment apparatus of the invention is able to effectively use an electromagnetic wave energy generated by the radio frequency current, which makes it unnecessary to form the radio frequency closed circuit including the organism tissue inside thereof, as it is indispensable in the conventional technique; and accordingly, a counter electrode plate as a diffusion electrode becomes unnecessary. And, effectively using the electromagnetic wave energy and concentrating in the local region of the organism tissue the local heat by the discharge between the discharge electrode and the organism tissue and the Joule heat by the current running locally through the organism tissue, the organism tissue treatment apparatus using a radio frequency facilitates to precisely applies a treatment to the organism tissue; and as a result, while a treatment is carried out to the local region of the organism tissue, the organism tissue treatment apparatus does not denature a periphery of the treated part of the organism tissue widely, does not flow the radio frequency current across the organism tissue either, and securely carries out various treatments to the organism tissue.
**(2)** Since the foregoing electromagnetic wave radiant electrode is connected to the main body of the organism tissue treatment apparatus only through the radio frequency current cable having a core wire and a shielded wire that coaxially encloses the core wire through an insulating material, the organism tissue treatment apparatus prevents a leakage of the electromagnetic wave from the radio frequency current cable to thereby enhance the transmission efficiency of the radio frequency energy, and the radio frequency current cable is able to effectively achieve the matching function as a matching cable.
**(3)** Since the foregoing radio frequency current cable is divided into a plurality of divided cables joined by a connector and of the divided cables that are adjoining each other, the divided cable on the front end side thereof is made smaller in the cable diameter than the divided cable on the main body of the organism tissue treatment apparatus, while seeking the matching adjustment across the total length of the matching cable, it is possible to decrease the electric resistance of the divided cable on the main body side of the organism tissue treatment apparatus as low as possible so as to restrain the energy loss of the radio frequency current as low as possible on one side, and to enhance the flexibility of the divided cable on the front end side thereof and enhance the capability to follow the probe during handling the probe and enhance the maneuverability of the probe on the other side.
**(4)** The foregoing electromagnetic wave radiant electrode is held by the grip of the probe, the tip of the electromagnetic wave radiant electrode is projected from the front end of the grip, the electromagnetic wave radiant electrode is connected to the active output terminal inside the grip, and
   **(4a)** either the passive output terminal is kept in an open state; and therefore, the electromagnetic wave radiated from the active output terminal and an electromagnetic wave radiant electrode is not diffused as it radiates naturally in the air and is captured by the passive output terminal, and the diffusion of a radio frequency energy into the air can be prevented so as to effectively recover the radio frequency energy,
   **(4b)** or the electromagnetic wave radiant electrode is connected to the passive output terminal through an impedance circuit including at least one of at least one capacitor and at least one inductor; and therefore, the radio frequency characteristic of the radio frequency current flowing through the electromagnetic wave radiant electrode can be varied in accordance with the construction of the impedance circuit between the electromagnetic wave radiant electrode and the passive output terminal, and the optimum radio frequency characteristic can be chosen in accordance with the requirements according to the treatment to the organism tissue.
**(5)** Since the probe used in the foregoing organism tissue treatment apparatus possesses the grip and the electromagnetic wave radiant electrode projected from the front end of the grip, in which the axis of the grip is set at an offset position from the axis of the electromagnetic wave radiant electrode, in such an extent that the grip and the finger of an operator holding the grip do not obstruct the line of sight viewing the tip of the electromagnetic wave radiant electrode from the backward on the axis of the electromagnetic wave radiant electrode, the operator is able to maneuver the probe securely without the line of sight on the axis of the electromagnetic wave radiant electrode being obstructed by the grip or the finger of the operator holding the grip, and to carry out a treatment to the organism tissue precisely by such a probe.
**(6)** Independently thereof or in correspondence therewith, the foregoing electromagnetic wave radiant electrode and an optional facing electrode that makes a pair with the electromagnetic wave radiant electrode to form a bi-polar electrode together with the electromagnetic wave radiant electrode are held by the grip of the probe, and both of the tips of the electromagnetic wave radiant electrode and the facing electrode are projected from the front end of the grip; and inside the grip, the electromagnetic wave radiant electrode is connected to the active output terminal, and on the other hand, the foregoing facing electrode is connected to the passive output terminal; and therefore, in the same manner as in the mono-polar probe, the electromagnetic wave radiant electrode radiates a strong electromagnetic wave from the tip thereof, and when the tip of the electromagnetic wave radiant electrode is approached to the local region of the organism tissue, until the organism tissue is exposed in the electromagnetic field near the tip of the electromagnetic wave radiant electrode, an arc discharge is generated between the tip of the electromagnetic wave radiant electrode and the local region of the organism tissue, and at the same time, an arc current flows into the tip of the facing electrode through the organism tissue to locally generate a Joule heat in the local region of the organism tissue. In consequence, a treatment such as an evaporation and dissection, etc., can be applied to the local region of the organism tissue, and a treatment such as a coagulation and hemostasis, etc., can be applied effectively by utilizing the thermal denaturation generated in the local region of the organism tissue.

## Claims

1. An organism tissue treatment apparatus using a radio frequency, comprising
- a probe (4) having an electrode grip (4a),
- an electromagnetic wave radiant electrode (5) held by the electrode grip (4a) of the probe (4), having an electrode tip on a front end side thereof, supplied with a radio frequency current having a high band radio frequency from eight Megahertz to sixty Megahertz or higher,
- a radio frequency current supply means (1) that supplies the radio frequency current to the electromagnetic wave radiant electrode (5), and
- a radio frequency current cable (11)
-- having a core wire (2) and a shielded wire (6) that coaxially encloses the core wire (2) through an insulating material and
-- the cable (11) being divided into a plurality of divided cables (11a, 11b) joined by a connector (13)
-- the cable diameter of the divided cable (11b) on the side of the electrode tip being made smaller than the cable diameter of the divided cable (11a) on the main body of the organism tissue treatment apparatus,
- wherein the radio frequency current cable (11) directly transmits the radio frequency current to the electromagnetic wave radiant electrode (5),
- wherein the radio frequency current is transmitted through a matching unit (3) and through the radio frequency current cable (11) from the radio frequency current supply means (1), the matching unit (3) being arranged outside the probe (4), and
- wherein the electrode tip of the electromagnetic wave radiant electrode (5) comprises a tip surface that locally radiates an electromagnetic energy from the electrode tip in an electromagnetic field adjacent to the electrode tip, the electromagnetic field having a strength required for applying a tissue treatment including evaporation, dissection, coagulation, hemostasis, and the like to the organism tissue (8) exposed in the electromagnetic field in no contact with the electrode tip, accompanied by an arc discharge generated between the electrode tip of the electromagnetic wave radiant electrode (5) and a local region (9) of the organism tissue (8) as an earth, and a Joule heat generated by an arc discharge current flowing into the local region (9) of the organism tissue (8) as the earth.

2. An organism tissue treatment apparatus using a radio frequency according to claim 1,
- wherein the electromagnetic wave radiant electrode (5) is held by the electrode grip (4a) of the probe (4),
- wherein the electrode tip of the electromagnetic wave radiant electrode (5) is projected from a front end of the electrode grip (4a) of the probe (4), and
- wherein inside the electrode grip (4a), the electromagnetic wave radiant electrode (5) is connected to an active output terminal and is kept in an open state to a passive output terminal.

3. An organism tissue treatment apparatus using a radio frequency according to claim 1,
- wherein the electromagnetic wave radiant electrode (5) is held by the electrode grip (4a) of the probe (4),
- wherein the electrode tip of the electromagnetic wave radiant electrode (5) is projected from a front end of the electrode grip (4a) of the probe (4), and
- wherein inside the electrode grip (4a), the electromagnetic wave radiant electrode (5) is connected to an active output terminal and is connected to a passive output terminal through an impedance circuit including at least one of at least one capacitor and at least one inductor.

4. An organism tissue treatment apparatus using a radio frequency according to any of claims 1 to 3,
- wherein the electromagnetic wave radiant electrode (5) and a facing electrode that makes a pair with the electromagnetic wave radiant electrode (5) to form a bipolar electrode together with the electromagnetic wave radiant electrode (5) are held by the electrode grip (4a) of the probe (4),
- wherein both of the tips of the electromagnetic wave radiant electrode (5) and of the facing electrode are projected from the front end of the electrode grip (4a) of the probe (4), and
- wherein inside the electrode grip (4a), the electromagnetic wave radiant electrode (5) is connected to the active output terminal, and on the other hand, the facing electrode is connected to the passive output terminal.

5. An organism tissue treatment apparatus using a radio frequency according to any of claims 1 to 4,
- wherein the probe (4) has the electrode grip (4a) and the electromagnetic wave radiant electrode (5) projected from the front end of the electrode grip (4a), and
- wherein an axis of the electrode grip (4a) is set at an offset position from an axis of the electromagnetic wave radiant electrode (5), in such an extent that the electrode grip (4a) and a finger of an operator holding the electrode grip (4a) do not obstruct a line (16) of sight viewing the electrode tip of the electromagnetic wave radiant electrode (5) from a backward on the axis of the electromagnetic wave radiant electrode (5).

## Patentansprüche

1. Vorrichtung zur Hochfrequenz-Behandlung eines Organismusgewebes, die folgendes umfasst:
- eine Sonde (4) mit einem Elektrodengriff (4a),
- eine Elektromagnetwellen-Ausstrahlungselektrode (5), die von dem Elektrodengriff (4a) der Sonde (4) gehalten wird, mit einer Elektrodenspitze an ihrer vorderen Endseite, die mit einem Hochfrequenzstrom versorgt wird, der eine Breitband-Hochfrequenz von acht Megahertz bis sechzig Megahertz oder höher hat,
- eine Hochfrequenzstromversorgungseinrichtung (1), die der Elektromagnetwellen-Ausstrahlungselektrode (5) den Hochfrequenzstrom zuführt, und
- ein Hochfrequenzstromkabel (11)
-- mit einer Kernader (2) und einer geschirmten Ader (6), die die Kernader (2) durch ein Isoliermaterial koaxial umschließt, und
-- wobei das Kabel (11) in mehrere geteilte Kabel (11a, 11b) geteilt ist, die durch einen Verbinder (13) zusammengefügt sind,
-- wobei der Kabeldurchmesser des geteilten Kabels (11b) auf der Seite der Elektrodenspitze kleiner gemacht ist als der Kabeldurchmesser des geteilten Kabels (11a) auf dem Hauptkörper der Vorrichtung zum Behandeln eines Organismusgewebes,
- wobei das Hochfrequenzstromkabel (11) den Hochfrequenzstrom direkt zur Elektromagnetwellen-Ausstrahlungselektrode (5) überträgt,
- wobei der Hochfrequenzstrom von der Hochfrequenzstromversorgungseinrichtung (1) durch eine Abgleichungseinheit (3) und durch das Hochfrequenzstromkabel (11) übertragen wird, wobei die Abgleichungseinheit (3) außerhalb der Sonde (4) angeordnet ist, und
- wobei die Elektrodenspitze der Elektromagnetwellen-Ausstrahlungselektrode (5) eine Spitzenoberfläche umfasst, die lokal elektromagnetische Energie aus der Elektrodenspitze in ein elektromagnetisches Feld benachbart der Elektrodenspitze ausstrahlt, wobei das elektromagnetische Feld eine Stärke aufweist, die erforderlich ist zur Anwendung einer Gewebebehandlung einschließlich Verdampfen, Dissektion, Koagulation, Hämostase und dergleichen auf das Organismusgewebe (8), das dem elektromagnetischen Feld ohne Kontakt mit der Elektrodenspitze ausgesetzt ist, zusammen mit einer Bogen, die entladung zwischen der Elektrodenspitze der Elektromagnetwellen-Ausstrahlungselektrode (5) und einem lokalen Bereich (9) des Organismusgewebes (8) als Masse erzeugt wird, und einer jouleschen Wärme, die durch einen Bogenentladungsstrom, der in den lokalen Bereich (9) des Organismusgewebes (8) als Masse fließt, erzeugt wird.

2. Vorrichtung zur Hochfrequenz-Behandlung eines Organismusgewebes nach Anspruch 1,
- wobei die Elektromagnetwellen-Ausstrahlungselektrode (5) von dem Elektrodengriff (4a) der Sonde (4) gehalten wird,
- wobei die Elektrodenspitze der Elektromagnetwellen-Ausstrahlungselektrode (5) von einem Vorderende des Elektrodengriffs (4a) der Sonde (4) projiziert wird, und
- wobei im Inneren des Elektrodengriffs (4a) die Elektromagnetwellen-Ausstrahlungselektrode (5) mit einem aktiven Ausgabeanschluss verbunden ist und in einem geöffneten Zustand zu einem passiven Ausgabeanschluss gehalten wird.

3. Vorrichtung zur Hochfrequenz-Behandlung eines Organismusgewebes nach Anspruch 1,
- wobei die Elektromagnetwellen-Ausstrahlungselektrode (5) von dem Elektrodengriff (4a) der Sonde (4) gehalten wird,
- wobei die Elektrodenspitze der Elektromagnetwellen-Ausstrahlungselektrode (5) von einem Vorderende des Elektrodengriffs (4a) der Sonde (4) projiziert wird, und
- wobei im Inneren des Elektrodengriffs (4a) die Elektromagnetwellen-Ausstrahlungselektrode (5) mit einem aktiven Ausgabeanschluss verbunden ist und durch eine Impedanzschaltung, die zumindest einen von mindestens einem Kondensator und mindestens einem Induktor aufweist, mit einem passiven Ausgabeanschluss verbunden ist.

4. Vorrichtung zur Hochfrequenz-Behandlung eines Organismusgewebes nach einem der Ansprüche 1 bis 3,
- wobei die Elektromagnetwellen-Ausstrahlungselektrode (5) und eine gegenüberliegende Elektrode, die mit der Elektromagnetwellen-Ausstrahlungselektrode zur Bildung einer bipolaren Elektrode zusammen mit der Elektromagnetwellen-Ausstrahlungselektrode (5) ein Paar ergibt, von dem Elektrodengriff (4a) der Sonde (4) gehalten werden,
- wobei beide Spitzen der Elektromagnetwellen-Ausstrahlungselektrode (5) und der gegenüberliegenden Elektrode von dem Vorderende des Elektrodengriffs (4a) der Sonde (4) vorstehen, und
- wobei im Inneren des Elektrodengriffs (4a) die Elektromagnetwellen-Ausstrahlungselektrode (5) mit dem aktiven Ausgabeanschluss verbunden ist und die gegenüberliegende Elektrode andererseits mit dem passiven Ausgabeanschluss verbunden ist.

5. Vorrichtung zur Hochfrequenz-Behandlung eines Organismusgewebes nach einem der Ansprüche 1 bis 4,
- wobei bei der Sonde (4) der Elektrodengriff (4a) und die Elektromagnetwellen-Ausstrahlungselektrode (5) von dem Vorderende des Elektrodengriffs (4a) vorstehen, und
- wobei eine Achse des Elektrodengriffs (4a) an eine von einer Achse der Elektromagnetwellen-Ausstrahlungselektrode (5) in einem solchen Ausmaß versetzte Position gesetzt ist, dass der Elektrodengriff (4a) und ein Finger einer Bedienperson, die den Elektrodengriff (4a) hält, eine Sichtlinie (16), in der die Elektrodenspitze der Elektromagnetwellen-Ausstrahlungselektrode (5) von hinten auf der Achse der Elektromagnetwellen-Ausstrahlungselektrode (5) gesehen wird, nicht verdecken.

## Revendications

1. Dispositif de traitement de tissu organique utilisant une radiofréquence, comprenant :
- une sonde (4) comportant un porte-électrode (4a),
- une électrode rayonnant une onde électromagnétique (5) maintenue par le porte-électrode (4a) de la sonde (4), comportant une extrémité d'électrode d'un côté d'extrémité avant de celle-ci, recevant un courant radiofréquence ayant une fréquence radio de bande élevée de 8 MHz à 60 MHz ou plus,
- des moyens d'alimentation en courant radiofréquence (1) qui alimentent l'électrode rayonnant une onde électromagnétique (5) en courant radiofréquence, et
- un câble de courant radiofréquence (11)
-- comportant un fil central (2) et un fil de blindage (6) qui enferme coaxialement le fil central (2) par l'intermédiaire d'un matériau isolant, et
-- le câble (11) étant divisé en une pluralité de câbles divisés (11a, 11b) raccordés par un connecteur (13),
- le diamètre de câble du câble divisé (11b) du côté de l'extrémité d'électrode étant plus petit que le diamètre de câble du câble divisé (11a) sur le corps principal du dispositif de traitement de tissu organique,
- dans lequel le câble de courant radiofréquence (11) transmet directement le courant radiofréquence à l'électrode rayonnant une onde électromagnétique (5),
- dans lequel le courant radiofréquence est transmis par les moyens d'alimentation en courant radiofréquence (1) par l'intermédiaire d'une unité d'adaptation (3) et par l'intermédiaire du câble de courant radiofréquence (11), l'unité d'adaptation (3) étant agencée à l'extérieur de la sonde (4), et
- dans lequel l'extrémité d'électrode de l'électrode rayonnant une onde électromagnétique (5) comprend une surface d'extrémité qui rayonne localement une énergie électromagnétique à partir de l'extrémité d'électrode dans un champ électromagnétique adjacent à l'extrémité d'électrode, le champ électromagnétique ayant une intensité nécessaire pour appliquer un traitement de tissu comprenant une évaporation, une dissection, une coagulation, une hémostase et similaire au tissu organique (8) exposé dans le champ électromagnétique sans contact avec l'extrémité d'électrode, accompagné d'une décharge en arc générée entre l'extrémité d'électrode de l'électrode rayonnant une onde électromagnétique (5) et une région locale (9) du tissu organique (8) en tant que terre, et d'un effet Joule généré par un courant de décharge en arc circulant dans la région locale (9) du tissu organique (8) en tant que terre.

2. Dispositif de traitement de tissu organique utilisant une radiofréquence selon la revendication 1,
- dans lequel l'électrode rayonnant une onde électromagnétique (5) est maintenue par le porte-électrode (4a) de la sonde (4),
- dans lequel l'extrémité d'électrode de l'électrode rayonnant une onde électromagnétique (5) fait saillie d'une extrémité avant du porte-électrode (4a) de la sonde (4), et
- dans lequel, à l'intérieur du porte-électrode (4a), l'électrode rayonnant une onde électromagnétique (5) est connectée à une borne de sortie active et est maintenue dans un état ouvert à une borne de sortie passive.

3. Dispositif de traitement de tissu organique utilisant une radiofréquence selon la revendication 1,
- dans lequel l'électrode rayonnant une onde électromagnétique (5) est maintenue par le porte-électrode (4a) de la sonde (4),
- dans lequel l'extrémité d'électrode de l'électrode rayonnant une onde électromagnétique (5) fait saillie d'une extrémité avant du porte-électrode (4a) de la sonde (4), et
- dans lequel, à l'intérieur du porte-électrode (4a), l'électrode rayonnant une onde électromagnétique (5) est connectée à une borne de sortie active et est connectée à une borne de sortie passive par l'intermédiaire d'un circuit d'impédance comprenant au moins l'un d'au moins un condensateur et d'au moins une inductance.

4. Dispositif de traitement de tissu organique utilisant une radiofréquence selon l'une quelconque des revendications 1 à 3,
- dans lequel l'électrode rayonnant une onde électromagnétique (5) et une électrode en regard qui forme une paire avec l'électrode rayonnant une onde électromagnétique (5) pour former une électrode bipolaire avec l'électrode rayonnant une onde électromagnétique (5) sont maintenues par le porte-électrode (4a) de la sonde (4),
- dans lequel les deux extrémités de l'électrode rayonnant une onde électromagnétique (5) et de l'électrode en regard font saillie de l'extrémité avant du porte-électrode (4a) de la sonde (4), et
- dans lequel, à l'intérieur du porte-électrode (4a), l'électrode rayonnant une onde électromagnétique (5) est connectée à la borne de sortie active et, d'autre part, l'électrode en regard est connectée à la borne de sortie passive.

5. Dispositif de traitement de tissu organique utilisant une radiofréquence selon l'une quelconque des revendications 1 à 4,
- dans lequel la sonde (4) comporte le porte-électrode (4a) et l'électrode rayonnant une onde électromagnétique (5) faisant saillie de l'extrémité avant du porte-électrode (4a), et
- dans lequel un axe du porte-électrode (4a) est positionné à une position décalée par rapport à un axe de l'électrode rayonnant une onde électromagnétique (5), dans une mesure telle que le porte-électrode (4a) et un doigt d'un opérateur tenant le porte-électrode (4a) n'obstruent pas une ligne (16) de vision de l'extrémité d'électrode de l'électrode rayonnant une onde électromagnétique (5) à partir de l'arrière sur l'axe de l'électrode rayonnant une onde électromagnétique (5).
